Europäisches Patentamt

European Patent Office

Office européen des brevets

(19)

(11) **EP 1 049 649 B1**

(12) **FASCICULE DE BREVET EUROPEEN**

(45) Date de publication et mention
de la délivrance du brevet:
**14.05.2003 Bulletin 2003/20**

(21) Numéro de dépôt: **99900968.1**

(22) Date de dépôt: **20.01.1999**

(51) Int Cl.7: **C01B 33/193**

(86) Numéro de dépôt international:
**PCT/FR99/00110**

(87) Numéro de publication internationale:
**WO 99/036360 (22.07.1999 Gazette 1999/29)**

(54) **MICROPERLES DE SILICE A PROPRIETE SENSORIELLE EN BOUCHE, LEUR PROCEDE DE PREPARATION ET COMPOSITIONS DENTIFRICES LES CONTENANT**

KIESELSAÜREMIKROKUGELN MIT SENSORISCHEN EIGENSCHAFTEN IM MUND , VERFAHREN ZU IHRER HERSTELLUNG UND ZAHNPASTEN DIE SIE ENTHALTEN

SILICA MICROBEADS HAVING SENSORY PROPERTIES IN THE MOUTH, THEIR METHOD OF PRODUCTION AND TOOTHPASTE PRODUCTS CONTAINING SAME

(84) Etats contractants désignés:
**AT BE CH CY DE DK ES FI FR GB GR IE IT LI LU NL PT SE**

(30) Priorité: **20.01.1998 FR 9800564**

(43) Date de publication de la demande:
**08.11.2000 Bulletin 2000/45**

(73) Titulaire: **RHODIA CHIMIE**
**92408 Courbevoie Cédex (FR)**

(72) Inventeurs:
• **AMICHE, Frédéric**
**F-92420 Vaucresson (FR)**

• **DROMARD, Adrien**
**F-69006 Lyon (FR)**
• **CHEVALLIER, Yvonnick**
**F-69270 Fontaines-Saint-Martin (FR)**
• **LAHARY, Pierre-Yves**
**F-69006 Lyon (FR)**

(74) Mandataire: **Fabre, Madeleine-France**
**Rhodia Services,**
**Direction de la Propriété Industrielle,**
**40, rue de la Haie-Coq**
**93306 Aubervilliers Cedex (FR)**

(56) Documents cités:
**WO-A-96/30302          FR-A- 2 632 185**

**Description**

La présente invention a pour objet une silice sous forme de microperles susceptibles de se désintégrer au sein d'une composition dentifrice les contenant, lors d'une opération de brossage, apportant ainsi un effet sensoriel en bouche, son procédé de préparation, son utilisation comme additif sensoriel dans les compositions dentifrices et les compositions dentifrices contenant ladite silice sous forme de microperles.

Les charges minérales utilisées dans les pâtes dentifrices ont généralement des tailles de particules inférieures à 50μm et n'apportent pas d'effet sensoriel spécifique.

L'idée d'introduire dans les pâtes dentifrices des particules minérales de grosse taille (au moins 100μm) et de cohésion limitée, apportant un effet sensoriel par désintégration au brossage est connu.

Le brevet US-A-4,871,396 décrit l'introduction dans les pâtes dentifrices de granules se désintégrant au brossage, obtenues par atomisation d'une dispersion aqueuse d'au moins deux poudres inorganiques (zéolite et silice colloïdale en particulier), la silice colloïdale ayant la fonction d'un liant.

La demande de brevet GB-A-2 272 640 décrit des granules de 0,03 à 3 mm à base de silice de précipitation épaississante obtenues de préférence par granulation et leur utilisation dans les pâtes dentifrices ; lesdites granules sont friables dans les conditions d'usage de la pâte dentifrice.

Les demandes de brevet WO 96/09033 et WO 96/09034 décrivent des granules de 40 à 600 μm obtenues par granulation d'un mélange de deux types de silice, de la silice dite abrasive d'une part et de la silice dite épaississante d'autre part, et l'utilisation de ces granules dans les pâtes dentifrices ; lesdites granules se désintègrent au brossage en apportant des bénéfices perceptibles d'une manière sensorielle ; la cohésion des granules est ajustée par le rapport silice abrasive / silice épaississante.

Des microperles de silice de précipitation définie par des caractéristiques de surface spécifique BET et CTAB d'au moins 100 m$^2$/g et une prise d'huile DOP d'au moins 200ml/100 g, obtenues par atomisation sont connues comme charges renforçantes dans les élastomères.

L'obtention de ces microperles nécessite la mise en oeuvre, pour la réalisation de l'opération d'atomisation, d'une suspension de silice à haute teneur en extrait sec, généralement d'au moins 15%. De tels extraits secs sont généralement obtenus par compactage des gâteaux de filtration et remise en suspension (appelée fluidification ou délitage) pour obtenir un niveau de viscosité compatible avec une opération d'atomisation.

Il a été proposé de réaliser cette opération de fluidification ou délitage par broyage humide (FR-A-2 453 880), par acidification (FR-A-2 453 880) ou par addition de sels d'aluminium (FR-A-2 536380).

Le délitage par broyage humide présente l'inconvénient d'une productivité insuffisante et d'un manque de stabilité des suspensions délitées à la sortie du broyeur. Le délitage par acidification conduit à des microperles de cohésion difficilement contrôlable ; celui par addition de sels d'aluminium conduit à des microperles réagissant avec les ions fluorures et autres agents anti-caries présents dans les dentifrices.

En outre ces microperles présentent l'inconvénient de présenter un faible indice de réfraction et ainsi de ne pas être suffisamment visibles dans les compositions dentifrices.

Il est connu de traiter ou de fonctionnaliser la surface des particules de silice à l'aide de dérivés hydrosolubles du zinc afin de modifier les interactions ou la réactivité de celle-ci avec le milieu dans lequel elle se trouve formulée.

Ainsi la demande de brevet FR-A-2 632 185 décrit la modification par traitement avec un zincate alcalin de silice précipitée abrasive ou épaississante de faible granulométrie (de 1 à 10 μm) afin d'améliorer la compatibilité de la silice avec des formulations dentifrices contenant des dérivés du zinc.

Le brevet EP-A-356 406 décrit la modification, par du chlorure de zinc, de la surface de charges pour papier constituées de particules formées d'un coeur de carbonate de calcium et d'une coquille de silice, ce afin de les rendre insensibles aux milieux acides.

Les demandes de brevets WO96/30302 et WO96/30301 visent des microperles de silice de grosse granulométrie, utilisées pour le renforcement des élastomères, lesdites microperles ayant été modifiées par un traitement à l'aide d'un composé du zinc (sulfate de zinc) en quantité élevée (1 à 5 parties en poids exprimé en Zn métal pour 100 parties en poids de silice SiO$_2$), suivi d'une étape de délitage réalisée de préférence en présence d'un sel d'aluminium. En formulation dentifrice, de telles microperles présenteraient les inconvénients d'une visibilité insuffisante en raison de leur faible indice de réfraction, d'une mauvaise compatibilité avec les ions fluorures en raison de la présence préférentielle de sel d'aluminium.

La Demanderesse a trouvé des microperles de silice non ou faiblement abrasive, qui en raison de leur meilleur facteur de forme (particules sphériques, peu ou pas angulaires, de répartition de taille homogène) et de leur cohésion contrôlée amenée par la présence en surface d'une faible quantité d'un dérivé du zinc, apporte dans les pâtes dentifrices un effet sensoriel en bouche spécifique amélioré, lors du brossage, par rapport à celui conféré par des granules de silice épaississante de même diamètre ou par des granules de mélange de silice abrasive et épaississante. En outre ces microperies sont compatibles avec les autres ingrédients de la formulation dentifrice.

Par ailleurs, le procédé de préparation par précipitation mis en oeuvre permet l'ajout au cours de la synthèse, de pigments, blancs notamment, tels que le dioxyde de titane ou l'oxyde de zinc, améliorant la visibilité des microperles de silice dans la pâte dentifrice et ce sans dégradation de la cohésion desdites microperles.

Un premier objet de l'invention consiste en des microperles de silice de précipitation, utilisables comme additif sensoriel dans les compositions dentifrices, présentant :

* une surface spécifique CTAB d'au moins 100 $m^2/g$, de préférence de l'ordre de 120 à 250 $m^2/g$, tout particulièrement de l'ordre de 140 à 200 $m^2/g$.
* une prise d'huile DOP d'au moins 200 ml/g, de préférence de l'ordre de 200 à 350 ml/g
* un diamètre médian de particule de 50 à 600$\mu$m, généralement de l'ordre de 100 à 400$\mu$m
* en surface de 0,5 à 2 parties en poids, de préférence de 0,5 à moins de 2 parties en poids, tout particulièrement de 0,6 à 1,5 partie en poids (exprimée en zinc métal) d'un dérivé du zinc à un degré d'oxydation "2" pour 100 parties en poids de silice ($SiO_2$).
* un pH de l'ordre de 7 à 9, de préférence de l'ordre de 7,5 à 8,7.

La surface spécifique CTAB est la surface externe déterminée selon la norme NFT 45007 (novembre 1987).

La prise d'huile DOP est déterminée selon la norme ISO 787/5 en mettant en oeuvre du dioctylphtalate.

La diamètre médian en poids $d_{50}$ des particules de silice est déterminé à l'aide d'un appareil SYMPATEC HELOS. Cet appareil applique le principe de la diffraction FRAUNHOFFER et met en oeuvre un laser He/Ne de faible puissance. L'échantillon est préalablement dispersé par agitation mécanique, sans application d'ultrasons, dans l'eau pendant 30 secondes pour obtenir une suspension aqueuse.

L'analyse granumométrique est aussi effectuée par tamisage à sec selon la norme NF X 11-507.

Le pH est mesuré selon la norme ISO 787/9 (pH d'une suspension à 5% en poids dans l'eau désionisée)

Lesdites microperles présentent en outre une surface spécifique BET d'au moins 100 $m^2/g$, de préférence de l'ordre de 120 à 300 $m^2/g$, tout particulièrement de l'ordre de 140 à 250 $m^2/g$.

La surface spécifique BET est déterminée selon la méthode de BRUNAUER - EMMET - TELLER décrite dans "The journal of the American Chemical Society", Vol. 60, page 309, février 1938 et correspondant à la norme ISO 5794/1 (annexe D).

Les microperles de silice de l'invention présentent la propriété de se désintégrer de manière adéquate au sein d'une composition dentifrice lors d'une opération de brossage, apportant ainsi un effet sensoriel en bouche.

Un mode d'évaluation de cet effet peut être la quantification de la cohésion des microperles de silice à l'aide d'un test spécifique de cohésion par agitation mécanique.

Ce test permet d'évaluer l'évolution du diamètre médian $d_{50}$ d'une suspension de silice, par mesure granulométrique avant et après 10 minutes d'agitation mécanique (sans mise en oeuvre d'ultrasons).

Selon ce test, la mesure granulométrique (par diffraction laser sur un granulomètre LASER SYMPATEC HELOS), est effectuée sur une suspension de silice présentant une concentration optique de 20% $\pm$ 3%, agitée mécaniquement (sans mise en oeuvre d'ultrasons).

Après 30s de dispersion mécanique de la suspension dans la cuve du granulomètre, on mesure le diamètre médian initial $d_{50i}$.

Après 10 minutes d'agitation, le niveau de la cuve ayant été éventuellement réajusté pour conserver une concentration optique de 20% $\pm$ 3% de la dispersion, on mesure le diamètre médian final $d_{50f}$.

Plus les diamètres après agitation mécanique demeurent proches des diamètres initiaux, plus les particules sont cohésives.

Les résultats seront d'autant plus comparatifs que les tailles des particules initiales seront proches. Pour cela on opérera si nécessaire un tamisage des particules.

Le facteur de cohésion FC, exprimé en %, basé sur les diamètres médians $d_{50}$ initial ($d_{50i}$) et $d_{50}$ final ($d_{50f}$) est calculé selon l'équation suivante :

$$FC = (d_{50f}/d_{50i}) \times 100$$

Selon ce test, décrit plus loin de manière plus détaillée, effectué sur un produit frais, les microperles de l'invention présentent, pour un diamètre médian initial de particule $d_{50i}$ de l'ordre de 180$\mu$m $\pm$10$\mu$m, un facteur de cohésion de 50 à moins de 90%, plus généralement de 55 à 80%.

Ces microperies présentent en outre la propriété d'être compatibles avec les additifs fluorés (NaF, monofluorophosphates, fluorures d'amines, ...) généralement présents dans les pâtes dentifrices ; leur compatibilité est supérieure à 75%, de préférence supérieure à 85% avec NaF.

La compatibilité des microperles (dénommées "silice" dans les tests) avec le fluorure de sodium (NaF) peut être mesurée selon un test dont le principe consiste :

- à laisser en contact la silice à tester avec une solution aqueuse de fluorure de sodium et de phosphates de concentration connue, pendant 1 heure à 60°C.
- à mesurer par ionométrie la concentration en ion fluorure [F⁻ non réagi] présent dans le milieu liquide obtenu par centrifugation
- et à comparer cette concentration à la concentration en ion fluorure [F⁻ de départ] de la solution initiale n'ayant eu aucun contact avec la silice.

La compatibilité avec NaF, exprimée en %, est obtenue par le calcul suivant :

$$( \text{[F}^- \text{ non réagi]} / \text{[F}^- \text{ de départ]} ) \times 100$$

Ce test est décrit plus loin de manière plus détaillée.

D'une manière préférentielle, lesdites microperles de silice renferment en outre au moins un pigment minéral, blanc notamment, comme le dioxyde de titane, l'oxyde de zinc... en quantité de l'ordre de 0,2 à 5 parties en poids, de préférence de 0,5 à 4 parties en poids (exprimées en poids de pigment, oxyde métallique en particulier) pour 100 parties en poids de silice ($SiO_2$).

Lesdites microperles sont susceptibles d'être obtenues selon un procédé par précipitation, comportant les étapes de formation de bouillie aqueuse de silice par réaction d'un silicate de métal alcalin M, de rapport $SiO_2 / M_2O$ de l'ordre de 2 à 4, avec un agent acidifiant, de séparation de la bouillie de silice formée, de lavage, de fluidification (délitage) du gâteau de silice récupéré et de séchage, ledit procédé étant caractérisé en ce que :

- l'opération de délitage est réalisée sur un gâteau de silice présentant un extrait sec d'au moins environ 15% en poids, ledit gâteau résultant de la séparation d'une bouillie de silice traitée à l'aide de 0,5 à 2 parties en poids, de préférence de 0,5 à moins de 2 parties en poids, tout particulièrement de 0,6 à 1 partie en poids (exprimée en zinc métal) d'au moins un composé soluble acide ou basique du zinc à un degré d'oxydation 2 pour 100 parties en poids de silice ($SiO_2$), et amenée à l'aide d'un agent respectivement basique (dans le cas d'un traitement à l'aide d'au moins un composé acide du zinc) ou acide (dans le cas d'un traitement à l'aide d'au moins un composé basique du zinc), à une valeur pH de l'ordre de 7,5 à 9,5 , de préférence de l'ordre de 7,5 à 9
- et l'opération de séchage est réalisée par atomisation.

Le choix du silicate et de l'agent acidifiant pour réaliser l'étape de formation de bouillie de silice selon le procédé de l'invention, se fait d'une manière bien connue en soi.

Le silicate de métal alcalin est avantageusement un silicate de sodium ou de potassium. On peut citer tout particulièrement les silicates de sodium.

Ledit silicate est mis en oeuvre sous forme d'une solution aqueuse présentant une concentration exprimée en $SiO_2$, de l'ordre de 20 à 350 g/l, de préférence de l'ordre de 100 à 260 g/l.

On utilise généralement comme agent acidifiant un acide minéral fort tel que l'acide sulfurique, l'acide nitrique, l'acide chlorhydrique ou un acide organique comme l'acide acétique, l'acide formique ou l'acide carbonique. D'une manière préférentielle, il s'agit de l'acide sulfurique. Ce dernier peut être mis en oeuvre sous forme dilué ou concentré, de préférence sous forme d'une solution aqueuse présentant une concentration de l'ordre de 60 à 400 g/l.

L'étape de formation de bouillie de silice peut être réalisée selon diverses méthodes connues à une température d'au moins 60°C, de préférence de l'ordre de 70 à 98°C, tout particulièrement de 80 à 98°C.

Ainsi, cette étape peut être effectuée par neutralisation progressive d'un pied de cuve constitué d'une solution aqueuse de silicate de métal alcalin contenant éventuellement un électrolyte, par addition continue ou discontinue d'un acide. Un tel mode opératoire est décrit notamment aux exemples 1 des documents FR-A-1 054 175 et US-A-2 940 830.

Une autre méthode peut consister à introduire simultanément une solution de silicate de métal alcalin et d'acide sur un pied de cuve constitué par :

- de l'eau éventuellement additionnée d'un acide ou d'une base, de pH de l'ordre de 4 à 11
- ou une solution aqueuse de silicate de métal alcalin contenant éventuellement un électrolyte, éventuellement partiellement neutralisée par un acide, ou une suspension (bouillie) de silice, de pH de l'ordre de 6 à 9

en maintenant pendant l'introduction simultanée des réactifs un pH du milieu sensiblement constant de l'ordre de 7 à 9 puis à introduire, si désiré, un acide jusqu'à obtention d'un pH de l'ordre de 3 à 6. Cette dernière opération d'introduction d'acide a pour but de faciliter les opérations suivantes de filtration et de lavage ; celle-ci peut être supprimée en tout ou partie lorsque le traitement par le composé soluble du zinc est réalisée à l'aide d'un sel acide de zinc.

Parmi les électrolytes pouvant être présent dans le pied de cuve, on peut citer notamment les sels solubles des métaux alcalins ou alcalino-terreux, notamment le sel du métal du silicate de départ et de l'agent acidifiant, à savoir de préférence le sulfate de sodium dans le cas de la réaction d'un silicate de sodium avec l'acide sulfurique. La quantité d'électrolyte présente peut être de l'ordre de 0,1 à 1 mole de sel électrolyte de métal alcalin ou de l'ordre de 10 à 100 mmoles de sel électrolyte de métal alcalino-terreux par litre de pied de cuve. Dans le cas du sulfate de sodium, celle-ci peut aller jusqu'à 17g/l, de préférence jusqu'à 14g/l de pied de cuve.

Des modes opératoires de neutralisation par introduction simultanée de silicate et d'acide dans un pied de cuve sont notamment décrits dans EP-A-18 866 ; EP-A-396 450 ; EP-A520 862 ; FR-A-2 710 629 et EP-A-670 813.

Parmi les composés solubles du zinc de degré d'oxydation "2" mis en oeuvre pour réaliser l'opération de traitement, on peut citer :

- les composés acides comme les sels inorganiques ou organiques tels que les halogénures (chlorure), oxyhalogénures (oxychlorure), nitrate, sulfate, carboxylates (acétate)
- les composés basiques comme les zincates contenant les anions $ZnO_2^{2-}$, $HZnO_2^{-}$, $Zn_2O_4^{4-}$, $ZnO_4^{6-}$, plus particu-lièrement les zincates alcalins, notamment le zincate de sodium ; (le mode de préparation des zincates alcalins par réaction de solutions alcalines avec un oxyde de zinc est décrit dans FR-A-2 632 185).

Dans la pratique le composé acide ou basique du zinc peut être utilisé sous la forme d'une solution, en général aqueuse.

Le traitement à l'aide d'un composé acide du zinc de degré d'oxydation "2" est suivi ou accompagné de l'addition d'un agent basique comme l'ammoniaque, l'hydroxyde de sodium, les silicates alcalins..., de préférence en solution aqueuse.

Le traitement à l'aide d'un composé basique du zinc de degré d'oxydation "2" est suivi de l'addition d'un agent acide comme les acides nitrique, sulfurique, chlorhydrique, carbonique..., de préférence en solution aqueuse.

Ledit traitement à l'aide d'au moins un composé soluble du zinc et de l'agent basique ou acide de précipitation du dérivé du zinc, peut être réalisé soit sur la bouillie en cours de précipitation, soit en fin de précipitation et/ou après précipitation, et ce avant l'étape de filtration/lavage.

Un premier mode de réalisation de l'opération de traitement par le composé soluble du zinc, consiste à ajouter à une bouillie de silice de pH de l'ordre de 7 à 9, de préférence de l'ordre de 7,5 à 8,5, en fin et/ou après précipitation à une température de l'ordre de 15 à 95°C, une solution aqueuse de sel acide soluble de zinc de degré d'oxydation "2" (sulfate de zinc par exemple), puis un agent basique (soude par exemple), jusqu'à obtenir un pH de l'ordre de 7,5 à 9,5, de préférence de l'ordre de 7,5 à 9. Après mûrissement éventuel (par exemple de 5 minutes à 2 heures), la bouillie de silice traitée est ensuite filtrée, lavée jusqu'à obtenir un gâteau de silice présentant un extrait sec généralement d'au moins 15% en poids. Des microperles de silice sont ensuite obtenues par délitage mécanique et séchage par atomisation.

Un deuxième mode de réalisation de l'opération de traitement par le composé soluble du zinc, consiste à ajouter à une bouillie de silice en cours de précipitation, de pH de l'ordre de 7 à 9, de préférence de l'ordre de 7,5 à 8,5 , simultanément une solution de silicate et une solution de sel acide de zinc (sulfate par exemple). Après mûrissement éventuel (par exemple de 10 minutes à 1 heure), la bouillie de silice traitée est ensuite filtrée, lavée jusqu'à obtenir un gâteau de silice présentant un extrait sec généralement d'au moins 15% en poids. Des microperles de silice sont ensuite obtenues par délitage mécanique et séchage par atomisation.

D'une manière préférentielle, ledit traitement est réalisé sur une bouillie de silice selon le premier mode ci-dessus décrit, à l'aide d'un composé soluble acide du zinc, notamment à l'aide de sulfate de zinc en solution aqueuse.

L'étape de séparation de la bouillie traitée est réalisée par filtration ou par tout autre moyen ; cette opération est de préférence couplée avec une opération de lavage.

Les étapes de filtration /lavage de la bouillie traitée sont de préférence réalisées à l'aide d'un filtre presse et d'eau comme agent de lavage, ce afin d'obtenir un gâteau de silice d'extrait sec désiré, d'au moins 15% en poids, de préférence d'au moins 16% en poids.

L'étape de délitage (c'est-à-dire de fluidification) du gâteau peut être réalisée par simple action d'agitation mécanique à l'aide d'un mobile cisaillant, éventuellement avec ajout d'eau.

La Demanderesse a constaté, et cela constitue un des avantages de l'invention, que la présence de zinc à la surface de la silice permet une opération de délitage particulièrement aisée du gâteau de silice, et ce sans nécessiter la mise en oeuvre de moyens chimiques (addition de sel d'aluminium par exemple) incompatibles avec des applications en dentifrices.

L'étape d'atomisation est de préférence réalisée à l'aide d'un atomiseur à buse.

Les microperles ainsi obtenues présentent un aspect sphérique, et présentent un diamètre médian de l'ordre de 50 à 600µm, généralement de l'ordre de 100 à 400µm.

Un mode de préparation tout particulièrement performant des microperles faisant l'objet de l'invention, consiste à effectuer dans les conditions ci-dessus mentionnées,

\*   une opération de formation de bouillie de silice par réaction d'un silicate de métal alcalin et d'un agent acidifiant, selon les étapes suivantes :

- une première étape consistant à mettre en oeuvre un pied de cuve initial constitué d'eau, de silicate de métal alcalin et d'un sel électrolyte, la concentration en silicate de métal alcalin (exprimé en SiO$_2$) dans ledit pied de cuve pouvant aller jusqu'à 100 g/l.
- une deuxième étape consistant à neutraliser ledit pied de cuve par l'agent acidifiant jusqu'à obtention d'un pH du milieu réactionnel supérieur ou égal à environ 7, de préférence de l'ordre de 7 à 9,5, tout particulièrement de l'ordre de 7 à 8,5 ;
- une troisième étape consistant à introduire dans ledit pied de cuve neutralisé le silicate de métal alcalin en solution aqueuse et l'agent acidifiant, dans des conditions telles que le pH du milieu réactionnel reste sensiblement constant et supérieur ou égal à environ 7, de préférence de l'ordre de 7 à 9,5, tout particulièrement de l'ordre de 7 à 8,5 ;

\*   une opération de traitement de la bouillie obtenue, après mûrissement éventuel, par addition de 0,5 à 2 parties, de préférence de 0,5 à moins de 2 parties, tout particulièrement de 0,6 à 1,5 partie (exprimée en zinc métal) d'au moins un composé soluble acide du zinc de degré d'oxydation "2" pour 100 parties de silice, puis, après mûrissement éventuel, addition d'un agent basique, ce jusqu'à obtenir un pH du milieu réactionnel de l'ordre de 7,5 à 9,5 , de préférence de l'ordre de 7,5 à 9 et mûrissement éventuel ;

\*   éventuellement une opération d'ajustement de pH à une valeur de l'ordre de 7 à 8,5 par addition d'un agent acide et mûrissement éventuel ;

\*   une opération de séparation et de lavage de manière à obtenir un gâteau de silice présentant un extrait sec d'au moins 15% en poids, de préférence d'au moins 16% en poids, notamment à l'aide d'un filtre presse;

\*   une opération de délitage par des moyens d'agitation mécaniques ;

\*   et une opération de séchage par atomisation.

Une variante du procédé de préparation permettant de préparer les microperles de silice de l'invention, consiste à introduire à un moment quelconque de la synthèse de la bouillie de silice (soit dans le pied de cuve, soit en cours ou après précipitation de la silice) ou de préférence lors de l'étape de délitage du gâteau de silice contenant du zinc, de l'ordre de 0,2 à 5 parties en poids, de préférence de l'ordre de 0,5 à 4 parties en poids pour 100 parties en poids de silice exprimées en SiO$_2$, d'au moins un pigment minéral, blanc notamment tel que le dioxyde titane ou l'oxyde de zinc.

La présente invention a également pour objet l'utilisation des microperles de silice faisant l'objet de l'invention ou obtenue selon le procédé de l'invention comme agent sensoriel en bouche, dans les compositions dentifrices, ainsi que les compositions dentifrices comprenant ladite silice.

Ladite silice peut être présente dans lesdites compositions dentifrices à raison de l'ordre de 0,5 à 20%, de préférence de l'ordre de 1 à 15% du poids desdites compositions.

Ces compositions peuvent en outre renfermer d'autres ingrédients habituels, en particulier des agents abrasifs minéraux, insolubles dans l'eau, des agents épaississants, des humectants...

Comme agents abrasifs, on peut mentionner en particulier les silices abrasives, le carbonate de calcium, l'alumine hydratée, la bentonite, le silicate d'aluminium, le silicate de zirconium, les métaphosphates et phosphates de sodium, de potassium, de calcium et de magnésium. La quantité totale de poudre(s) abrasive(s) peut constituer de l'ordre de 5 à 50% du poids de la composition dentaire.

Parmi les agents épaississants on peut mentionner tout particulièrement les silices épaississantes en quantité de l'ordre de 1 à 15% du poids, la gomme xanthane, la gomme guar, les carraghénanes, les dérivés de la cellulose, les alginates, en quantité pouvant aller jusqu'à 5% du poids de ladite composition....

Parmi les agents humectants on peut citer par exemple le glycérol, le sorbitol, les polyéthylène glycols, les polypropylène glycols, le xylitol, en quantité de l'ordre de 2 à 85%, de préférence de l'ordre de 3 à 55% du poids de composition dentifrice exprimé en sec.

Ces compositions dentifrices peuvent en outre comporter des agents tensio-actifs, des agents détergents, des colorants, des antibactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym...).

Les exemples suivants sont donnés à titre illustratif.

Mesure de la taille moyenne et de la cohésion des microperles

L'appareil utilisé est un granulomètre par diffraction laser SYMPATEC, muni d'un logiciel Helos, d'un système de dispersion liquide Sucell cl 245 et d'une focale d'analyse 500 mm. Pour toute mesure, la concentration optique de la suspension de silice dans la cuve du granulomètre est ajustée à 20 ±3%.

La granulométrie initiale des microperles, exprimée en diamètres $d_{10}$, $d_{50}$ et $d_{90}$, est mesurée après 30 secondes sous agitation mécanique à une vitesse correspondant à la position 8 de l'appareil.

La mesure de la cohésion des microsphères est réalisée avec le même appareillage, dans les mêmes conditions, après 5 minutes, 10 minutes, 15 minutes et 20 minutes d'agitation (la concentration optique de la suspension de silice dans la cuve du granulomètre étant, si nécessaire, ajustée à 20 ±3% par dilution). Les résultats sont également exprimés en diamètres $d_{10}$, $d_{50}$ et $d_{90}$

Plus les diamètres après agitation mécaniques demeurent proches des diamètres initiaux, plus les particules sont cohésives.

Les résultats étant d'autant plus comparatifs que les tailles des particules initiales sont proches, un tamisage des particules est effectué si nécessaire dans ce but.

Le facteur de cohésion FC, exprimé en %, est basé sur les diamètres médians $d_{50}$ initial ($d_{50i}$) et $d_{50}$ après 10 minutes ($d_{50f}$) et calculé selon l'équation suivante :

$$FC = (d_{50f}/d_{50i}) \times 100$$

Mesure de la compatibilité avec NaF :

- Préparation de la "solution de mesure" -

Une solution mère contenant 11,2 g de NaF, 86 g de $NaH_2PO_4,H_2O$, 333,6 g de $Na_2HPO_4,2H_2O$ et 2690 g d'eau permutée est d'abord préparée.

7g de silice et 30g de la solution mère préparée sont ensuite mis sous agitation à 60°C pendant 1 heure dans un flacon fermé hermétiquement.

Après refroidissement à température ambiante, le surnageant est récupéré après centrifugation.

La "solution de mesure" comprend enfin 10 ml de ce surnageant et 25 ml de tampon TAFIC de Radiometer Analytical S.A. (de pH = 5,3, constituée de NaCl 1M, CH3COOH 0.25M, CH3COONa 0.75M et acide cyclohexane diamine tétracétique 0.01M).

- Préparation de la "solution témoin" -

La "solution témoin" comprend 10 ml de la solution mère précédemment préparée et 25 ml de tampon TAFIC.

- Dosage -

Le dosage est effectué à l'aide d'une électrode spécifique aux ions fluor (Tacussel type PF4L1), d'une électrode de référence au Calomel (TR100) et d'un ionomètre (Tacussel type PHM 95).

La compatibilité avec NaF, exprimée en %, est obtenue par le calcul suivant :

$$([F^- \text{ non réagi}] / [F^- \text{ de départ}] ) \times 100$$

[F- non réagi] représentant la concentration en ion fluorure non réagi de la "solution de mesure"
[F- de départ] représentant la concentration en ion fluorure de la "solution témoin".

**Exemple 1**

Dans un réacteur en acier inoxydable, muni d'un système d'agitation par hélices et d'un chauffage à double enveloppe, on introduit :

- 660 litres d'eau
- 11,8 Kg de $Na_2SO_4$ (électrolyte)
- 323 litres de silicate de sodium aqueux, présentant un rapport pondéral $SiO_2$ / $Na_2O$ égal à 3,45 et une densité à 20°C égale à 1,230 .

La concentration en $SiO_2$ dans le pied de cuve est de 77g/l. Le mélange est porté à une température de 82°C tout en le maintenant sous agitation. On y introduit 395 litres d'acide sulfurique dilué de densité à 20°C égale à 1,050 jusqu'à obtenir dans le milieu réactionnel une valeur de pH (mesuré à sa température) égale à 7,5 . La température de réaction est de 82°C pendant les 15 premières minutes de la réaction ; elle est ensuite portée de 82°C à 95°C en 15 minutes environ, puis maintenue à 95°C jusqu'à la fin de la réaction.

On introduit ensuite conjointement dans le milieu de réaction 77 litres de silicate de sodium aqueux du type décrit ci-avant et 106 litres d'acide sulfurique, également du type décrit ci-avant, cette introduction simultanée d'acide et de silicate étant réalisée de manière telle que le pH du milieu de réaction, pendant la période d'introduction, soit constamment égal à 7,5 ± 0,1.

On introduit ensuite une solution aqueuse, contenant 85g/l de $ZnSO_4,7H_2O$, à un débit de 140 l/h pendant 12 minutes.

On procède ensuite à l'introduction d'une solution aqueuse contenant 180 g/l de NaOH de façon à amener la valeur du pH à 8. Quand cette valeur est atteinte on arrête l'introduction de la solution de NaOH et on laisse la bouillie réactionnelle 10 mn sous agitation. On introduit ensuite de l'acide sulfurique dilué de densité à 20°C égale à 1050 kg/$m^3$, de façon à amener la valeur du pH à 7. On maintient alors la bouillie réactionnelle à ce pH pendant 5 minutes. La durée totale de la réaction est de 126 minutes.

On obtient ainsi une suspension réactionnelle qui est filtrée et lavée au moyen d'un filtre presse de telle sorte que l'on récupère un gâteau de silice présentant un extrait sec de 19,4%.

Ce gâteau est ensuite fluidifié par action mécanique dans un déliteur équipé d'une agitation centrale à quatre bipâles et d'une agitation périphérique de type raclant. Lors de cette opération de fluidification on introduit dans le déliteur 1,7 kg d'oxyde de titane en poudre. Après cette opération de délitage on obtient un gâteau pompable de pH égal à 8,1 et de perte au feu égale à 80%; ce gâteau est séché au moyen d'un atomiseur à buses.

Les caractéristiques des microperles de silice obtenues sont les suivantes :

- teneur en Zn métal        0,5%
- surface spécifique B.E.T.        180 $m^2$/g
- surface spécifique C.T.A.B.        184 $m^2$/g
- prise d'huile D.O.P.        260 mt/100g
- pH        8,2
- Na2SO4        0,7%
- teneur en $TiO_2$        1,7%

Les caractéristiques granulométriques obtenues par tamisage à sec sont les suivantes:

| Ouverture des tamis en µm | 250 | 210 | 180 | 150 | 125 |
|---|---|---|---|---|---|
| Refus cumulés en %poids/poids | 3 | 15 | 35 | 56 | 76 |

Les caractéristiques granulométriques (détermination Laser) des particules obtenues sont les suivantes :

$d_{10}$ = 72µm
$d_{50}$ = 158µm
$d_{90}$ = 256µm.

Après tamisage à sec, les résultats du test de cohésion sont les suivants :

| Agitation mécanique durée | $d_{10}$ (µm) | $d_{50}$ (µm) | $d_{90}$ (µm) |
|---|---|---|---|
| 30 secondes | 85 | 178 | 277 |
| 5 minutes | 16 | 119 | 224 |
| 10 minutes | 13 | 100 | 203 |
| 15 minutes | 11 | 83 | 176 |
| 20 minutes | 10 | 77 | 168 |

- FC = 56,2%
- compatibilité avec NaF : 96%

**Exemple 2**

Dans un réacteur en acier inoxydable, muni d'un système d'agitation par hélices et d'un chauffage à double enveloppe, on introduit :

- 660 litres d'eau
- 11,8 Kg de $Na_2SO_4$ (électrolyte)
- 323 litres de silicate de sodium aqueux, présentant un rapport pondéral $SiO_2/Na_2O$ égal à 3,45 et une densité à 20°C égale à 1,230 .

La concentration en $SiO_2$ dans le pied de cuve est de 77g/l. Le mélange est porté à une température de 82°C tout en le maintenant sous agitation. On y introduit 395 litres d'acide sulfurique dilué de densité à 20°C égale à 1,050 jusqu'à obtenir dans le milieu réactionnel une valeur de pH (mesuré à sa température) égale à 7,5 . La température de réaction est de 82°C pendant les 15 premières minutes de la réaction ; elle est ensuite portée de 82°C à 95°C en 15 minutes environ, puis maintenue à 95°C jusqu'à la fin de la réaction.
On introduit ensuite conjointement dans le milieu de réaction 77 litres de silicate de sodium aqueux du type décrit ci-avant et 106 litres d'acide sulfurique, également du type décrit ci-avant, cette introduction simultanée d'acide et de silicate étant réalisée de manière telle que le pH du milieu de réaction, pendant la période d'introduction, soit constamment égal à 7,5 ± 0,1 .
On introduit ensuite une solution aqueuse, contenant 170 g/l de $ZnSO_4,7H_2O$, à un débit de 140l/h pendant 12 minutes. On procède ensuite à l'introduction d'une solution aqueuse contenant 180 g/l de NaOH de façon à amener la valeur du pH à 8. Quand cette valeur est atteinte on arrête l'introduction de la solution de NaOH et on laisse la bouillie réactionnelle 10 mn sous agitation. On introduit ensuite de l'acide sulfurique dilué de densité à 20°C égale à 1050 kg/$m^3$, de façon à amener la valeur du pH à 7. On maintient alors la bouillie réactionnelle à ce pH pendant 5 minutes. La durée totale de la réaction est de 128 minutes.
On obtient ainsi une suspension réactionnelle qui est filtrée et lavée au moyen d'un filtre presse de telle sorte que l'on récupère un gâteau de silice présentant un extrait sec de 20,1%.
Ce gâteau est ensuite fluidifié par action mécanique dans un déliteur équipé d'une agitation centrale à quatre bipâles et d'une agitation périphérique de type raclant. Lors de cette opération de fluidification on introduit dans le déliteur 1,7 kg d'oxyde de titane en poudre. Après cette opération de délitage on obtient un gâteau pompable de pH égal à 8,4 et de perte au feu égale à 79,5% ; ce gâteau est séché au moyen d'un atomiseur à buses. Les caractéristiques des microperles de silice obtenues sont les suivantes :

- teneur en Zn métal        1%
- surface spécifique B.E.T.        174 $m^2$/g
- surface spécifique C.T.A.B.        164 $m^2$/g
- prise d'huile D.O.P.        260 ml/100g
- pH        8,7
- $Na_2SO_4$        0.7%
- teneur en $TiO_2$        1,7%

Les caracteristiques granulometriques obtenues par tamisage à sec sont les suivantes:

| Ouverture des tamis en µm | 250 | 210 | 180 | 150 | 125 |
|---|---|---|---|---|---|
| Refus cumulés en %poids/poids | 7 | 21 | 43 | 65 | 81 |

Les caractéristiques granulométriques (détermination Laser) des particules obtenues sont les suivantes :

$d_{10}$ = 96 µm
$d_{50}$ = 174 µm
$d_{90}$ = 279 µm.

Les résultats du test de cohésion sont les suivants :

| Agitation mécanique durée | $d_{10}$ (µm) | $d_{50}$ (µm) | $d_{90}$ (µm) |
|---|---|---|---|
| 30 secondes | 96 | 174 | 279 |

(suite)

| Agitation mécanique durée | $d_{10}$ ($\mu$m) | $d_{50}$ ($\mu$m) | $d_{90}$ ($\mu$m) |
|---|---|---|---|
| 5 minutes | 61 | 147 | 244 |
| 10 minutes | 49 | 135 | 228 |
| 15 minutes | 42 | 125 | 213 |
| 20 minutes | 35 | 118 | 205 |

- FC = 77,6%
- compatibilité avec NaF : 90%

**Exemple 3**

Dans un réacteur en acier inoxydable, muni d'un système d'agitation par hélices et d'un chauffage à double enveloppe, on introduit :

- 660 litres d'eau
- 11,8 Kg de $Na_2SO_4$ (électrolyte)
- 323 litres de silicate de sodium aqueux, présentant un rapport pondéral $SiO_2$ / $Na_2O$ égal à 3,45 et une densité à 20°C égale à 1,230 .

La concentration en $SiO_2$ dans le pied de cuve est de 77g/l. Le mélange est porté à une température de 82°C tout en le maintenant sous agitation. On y introduit 395 litres d'acide sulfurique dilué de densité à 20°C égale à 1,050 jusqu'à obtenir dans le milieu réactionnel une valeur de pH (mesuré à sa température) égale à 7,5 . La température de réaction est de 82°C pendant les 15 premières minutes de la réaction ; elle est ensuite portée de 82°C à 95°C en 15 minutes environ, puis maintenue à 95°C jusqu'à la fin de la réaction.

On introduit ensuite conjointement dans le milieu de réaction 77 litres de silicate de sodium aqueux du type décrit ci-avant et 106 litres d'acide sulfurique, également du type décrit ci-avant, cette introduction simultanée d'acide et de silicate étant réalisée de manière telle que le pH du milieu de réaction, pendant la période d'introduction, soit constamment égal à 7,5 ± 0,1 .

On introduit ensuite une solution aqueuse, contenant 136 g/l de $ZnSO_4,7H_2O$, à un débit de 140 l/h pendant 12 minutes.
On procède ensuite à l'introduction d'une solution aqueuse contenant 180 g/l de NaOH de façon à amener la valeur du pH à 8. Quand cette valeur est atteinte on arrête l'introduction de la solution de NaOH et on laisse la bouillie réactionnelle 10 mn sous agitation. On introduit ensuite de l'acide sulfurique dilué de densité à 20°C égale à 1050 kg/ $m^3$, de façon à amener la valeur du pH à 7. On maintient alors la bouillie réactionnelle à ce pH pendant 5 minutes. La durée totale de la réaction est de 128 minutes.

On obtient ainsi une suspension réactionnelle qui est filtrée et lavée au moyen d'un filtre presse de telle sorte que l'on récupère un gâteau de silice présentant un extrait sec de 21,7%

Ce gâteau est ensuite fluidifié par action mécanique dans un déliteur équipé d'une agitation centrale à quatre bipâles et d'une agitation périphérique de type raclant. Lors de cette opération de fluidification on introduit dans le déliteur 1,7 kg d'oxyde de titane en poudre.

Après cette opération de délitage on obtient un gâteau pompable de pH égal à 8,2 et de perte au feu égale à 78,8%.
Ce gâteau est divisé en 2 parties.
La première partie est séchée au moyen d'un atomiseur à buses.
Les caractéristiques des microperles de silice obtenues sont les suivantes :

- teneur en Zn métal        0.8%
- surface spécifique B.E.T.        170 $m^2$/g
- surface spécifique C.T.A.B.        170 $m^2$/g
- prise d'huile D.O.P.        262 ml/100g
- pH        8.4
- $Na_2SO_4$        0.7%
- teneur en $TiO_2$        1,7%

Les caractéristiques granulométriques obtenues par tamisage à sec sont les suivantes:

| Ouverture des tamis en μm | 250 | 210 | 180 | 150 | 125 |
|---|---|---|---|---|---|
| Refus cumulés en %poids/poids | 4 | 19 | 43 | 57 | 79 |

Les caractéristiques granulométriques (détermination Laser) des particules obtenues sont les suivantes :

$d_{10}$ = 106μm
$d_{50}$ = 180μm
$d_{90}$ = 273μm.

Les résultats du test de cohésion sont les suivants :

| Agitation mécanique durée | $d_{10}$ (μm) | $d_{50}$ (μm) | $d_{90}$ (μm) |
|---|---|---|---|
| 30 secondes | 106 | 180 | 273 |
| 5 minutes | 45 | 141 | 236 |
| 10 minutes | 27 | 116 | 208 |
| 15 minutes | 20 | 101 | 189 |
| 20 minutes | 17 | 93 | 176 |

-  FC = 64,4%
-  compatibilité avec NaF : 95%

**Exemple 4**

La deuxième partie du gâteau préparé à l'exemple 3 est amenée à une perte au feu de 81,1% par ajout d'eau et maintenue sous agitation pendant 5 heures avant d'être séchée au moyen d'un atomiseur à buses.
Les caractéristiques des microperles de silice obtenues sont les suivantes :

-  teneur en Zn métal        0,8%
-  surface spécifique B.E.T.        171 $m^2$/g
-  surface spécifique C.T.A.B.        170 $m^2$/g
-  prise d'huile D.O.P.        266 ml/100g
-  pH        8,5
-  $Na_2SO_4$        0,7%
-  teneur en $TiO_2$        1,7%

Les caractéristiques granulométriques obtenues par tamisage à sec sont les suivantes:

| Ouverture des tamis en μm | 250 | 210 | 180 | 150 | 125 |
|---|---|---|---|---|---|
| Refus cumulés en %poids/poids | 2 | 9 | 29 | 54 | 74 |

Les caractéristiques granulométriques (détermination Laser) des particules obtenues sont les suivantes :

$d_{10}$ = 81 μm
$d_{50}$ = 157 μm
$d_{90}$ = 248 μm.

Après tamisage à sec, les résultats du test de cohésion sont les suivants :

| Agitation mécanique durée | $d_{10}$ (μm) | $d_{50}$ (μm) | $d_{90}$ (μm) |
|---|---|---|---|
| 30 secondes | 95 | 174 | 269 |
| 5 minutes | 37 | 128 | 224 |

(suite)

| Agitation mécanique durée | $d_{10}$ ($\mu$m) | $d_{50}$ ($\mu$m) | $d_{90}$ ($\mu$m) |
|---|---|---|---|
| 10 minutes | 24 | 108 | 202 |
| 15 minutes | 18 | 94 | 179 |
| 20 minutes | 16 | 87 | 171 |

- FC = 62,1%
- compatibilité avec NaF : 95%

**Exemple 5**

On répète l'opération décrite à l'exemple 3, en réalisant l'étape de traitement à l'aide d'une solution aqueuse contenant 85 g/l de $ZnSO_4,7H_2O$, à un débit de 560 l/h pendant 12 minutes, au lieu de 136 g/l de $ZnSO_4,7H_2O$, à un débit de 140l/h pendant 12 minutes.
Les caractéristiques des microperles de silice obtenues sont les suivantes :

- teneur en Zn métal        2%
- surface spécifique B.E.T.        170 $m^2$/g
- surface spécifique C.T.A.B.        150 $m^2$/g
- prise d'huile D.O.P.        258 ml/100g
- pH        8,6
- $Na_2SO_4$        0,8%

Les caractéristiques granulométriques obtenues par tamisage à sec sont les suivantes:

| Ouverture des tamis en $\mu$m | 250 | 210 | 180 | 150 | 125 |
|---|---|---|---|---|---|
| Refus cumulés en %poids/poids | 3 | 19 | 40 | 61 | 79 |

Les caractéristiques granulométriques (détermination Laser) des particules obtenues sont les suivantes :

$d_{10}$ = 85$\mu$m
$d_{50}$ = 173$\mu$m
$d_{90}$ = 275$\mu$m.

Les résultats du test de cohésion sont les suivants :

| Agitation mécanique durée | $d_{10}$ ($\mu$m) | $d_{50}$ (10$\mu$m) | $d_{90}$ ($\mu$m) |
|---|---|---|---|
| 30 secondes | 85 | 173 | 275 |
| 5 minutes | 77 | 164 | 259 |
| 10 minutes | 70 | 158 | 250 |
| 15 minutes | 64 | 153 | 245 |
| 20 minutes | 61 | 150 | 243 |

- FC = 91,3%
- compatibilité avec NaF : 98%

**Exemple 6 comparatif**

Dans un réacteur en acier inoxydable, muni d'un système d'agitation par hélices et d'un chauffage à double enveloppe, on introduit :

- 660 litres d'eau

- 11,8 Kg de $Na_2SO_4$ (électrolyte)
- 323 litres de silicate de sodium aqueux, présentant un rapport pondéral $SiO_2/Na_2O$ égal à 3,45 et une densité à 20°C égale à 1,230 .

La concentration en $SiO_2$ dans le pied de cuve est de 77g/l. Le mélange est porté à une température de 82°C tout en le maintenant sous agitation. On y introduit 395 litres d'acide sulfurique dilué de densité à 20°C égale à 1,050 jusqu'à obtenir dans le milieu réactionnel une valeur de pH (mesuré à sa température) égale à 7,5 . La température de réaction est de 82°C pendant les 15 premières minutes de la réaction ; elle est ensuite portée de 82°C à 95°C en 15 minutes environ, puis maintenue à 95°C jusqu'à la fin de la réaction.

On introduit ensuite conjointement dans le milieu de réaction 77 litres de silicate de sodium aqueux du type décrit ci-avant et 106 litres d'acide sulfurique; également du type décrit ci-avant, cette introduction simultanée d'acide et de silicate étant réalisée de manière telle que le pH du milieu de réaction, pendant la période d'introduction, soit constamment égal à 7,5 ± 0,1.

Après introduction de la totalité du silicate, on continue à introduire de l'acide dilué à un débit de 310 l/h , et ce pendant 5 minutes;

Cette introduction complémentaire d'acide amène alors le pH du milieu à une valeur égale à 5,0.

La durée totale de la réaction est fixée à 85 minutes.

On obtient ainsi une bouillie de silice précipitée, qui est filtrée et lavée au moyen d'un filtre presse, de telle sorte que l'on récupère finalement un gâteau de silice de perte au feu de 79% (soit un taux de matière sèche de 21% en poids).

Ce gâteau est ensuite fluidifié par action mécanique et chimique (ajout d'aluminate de sodium). Après cette opération de délitage, on obtient un gâteau pompable, de pH égal à 6,3 , qui est alors atomisé au moyen d'un atomiseur à buses. Les caractéristiques de la silice obtenue sont les suivantes :

- surface spécifique B.E.T.      170 $m^2/g$
- surface spécifique C.T.A.B.      160 $m^2/g$
- prise d'huile D.O.P.      300 ml/100g
- pH      6.7
- $Na_2SO_4$      1,2%

Les caractéristiques granulométriques obtenues par tamisage à sec sont les suivantes:

| Ouverture des tamis en μm | 250 | 210 | 180 | 150 | 125 |
|---|---|---|---|---|---|
| Refus cumulés en %poids/poids | 15 | 37 | 59 | 77 | 89 |

Les caractéristiques granulométriques (détermination Laser) des particules obtenues sont les suivantes :

$d_{10}$ = 63 μm
$d_{50}$ = 174 μm
$d_{90}$ = 285μm.

- compatibilité avec NaF : 39%

**Revendications**

**1.**  Silice de précipitation présentant :

- \*  une surface spécifique CTAB d'au moins 100 $m^2/g$, de préférence de l'ordre de 120 à 250 $m^2/g$, tout particulièrement de l'ordre de 140 à 200 $m^2/g$;
- \*  une prise d'huile DOP d'au moins 200 ml/g, de préférence de l'ordre de 200 à 350 ml/g ;
- \*  en surface un dérivé du zinc à un degré d'oxydation "2"
- \*  un pH de l'ordre de 7 à 9, de préférence de l'ordre de 7,5 à 8,7 ; ladite silice étant **caractérisée**
- \*  **en ce qu'**elle est sous forme de microperles présentant un diamètre médian de particule de 50 à 600μm, généralement de l'ordre de 100 à 400 μm,
- \*  **en ce que** la quantité de dérivé du zinc à un degré d'oxydation "2" présent en surface va de 0,5 à 2 parties en poids, de préférence de 0,5 à moins de 2 parties en poids, tout particulièrement de 0,6 à 1,5 partie en poids (exprimée en zinc métal) pour 100 parties en poids de silice ($SiO_2$),

\*   et **en ce que** lesdites microperles sont susceptibles de se désintégrer au sein d'une composition dentifrice les comprenant, lors d'une opération de brossage, apportant un effet sensoriel en bouche.

2.  Silice selon la revendication 1), **caractérisées en ce qu'**elles présente une surface spécifique BET d'au moins 100 m$^2$/g, de préférence de l'ordre de 120 à 300 m$^2$/g, tout particulièrement de l'ordre de 140 à 250 m$^2$/g.

3.  Silice selon la revendication 1) ou 2), **caractérisées en ce que** lesdites microperles présentent, pour un diamètre de 180μm ± 10μm, un facteur de cohésion de 50 à moins de 90%, de préférence de 55 à 80%.

4.  Silice selon l'une quelconque des revendications 1) à 3), **caractérisée en ce que** lesdites microperles sont compatibles avec les composés fluorés, leur compatibilité étant supérieure à 75%, de préférence supérieure à 85% avec NaF.

5.  Silice selon l'une quelconque des revendications 1) à 4), **caractérisée en ce que** lesdites microperles renferment au moins un pigment minéral, blanc notamment, en quantité de l'ordre de 0,2 à 5 parties en poids, de préférence de 0,5 à 4 parties en poids (exprimées en poids de pigment) pour 100 parties en poids de silice (SiO$_2$).

6.  Procédé de préparation de silice de précipitation sous forme de microperles à propriété sensorielle en bouche faisant l'objet de l'une quelconque des revendications 1) à 4), comportant les étapes de formation de bouillie aqueuse de silice par réaction d'un silicate de métal alcalin M, de rapport SiO$_2$ / M$_2$O de l'ordre de 2 à 4, avec un agent acidifiant, de séparation de la bouillie de silice formée, de lavage, de fluidification (délitage) du gâteau de silice récupéré et de séchage, ledit procédé étant **caractérisé en ce que** :

    -   l'opération de délitage est réalisée sur un gâteau de silice présentant un extrait sec d'au moins environ 15% en poids, ledit gâteau résultant de la séparation d'une bouillie de silice traitée à l'aide de 0,5 à 2 parties en poids, de préférence de 0,5 à moins de 2 parties en poids, tout particulièrement de 0,6 à 1 partie en poids (exprimée en zinc métal) d'au moins un composé soluble acide ou basique du zinc à un degré d'oxydation 2 pour 100 parties en poids de silice (SiO$_2$), et amenée à l'aide d'un agent respectivement basique (dans le cas d'un traitement à l'aide d'au moins un composé acide du zinc) ou acide (dans le cas d'un traitement à l'aide d'au moins un composé basique du zinc), à une valeur pH de l'ordre de 7,5 à 9,5 , de préférence de l'ordre de 7,5 à 9
    -   et l'opération de séchage est réalisée par atomisation.

7.  Procédé selon la revendication 6), **caractérisé en ce que** l'étape de formation de bouillie de silice est réalisée à une température d'au moins 60°C, de préférence de l'ordre de 70 à 98°C, tout particulièrement de 80 à 98°C.

8.  Procédé selon la revendication 6) ou 7), **caractérisé en ce que** l'étape de formation de bouillie est effectuée par neutralisation progressive d'un pied de cuve constitué d'une solution aqueuse de silicate de métal alcalin contenant éventuellement un électrolyte, par addition continue ou discontinue d'un acide.

9.  Procédé selon la revendication 6) ou 7), **caractérisé en ce que** l'étape de formation de bouillie est effectuée en introduisant simultanément une solution de silicate de métal alcalin et un acide sur un pied de cuve constitué par :

    -   de l'eau éventuellement additionnée d'un acide ou d'une base, de pH de l'ordre de 4 à 11
    -   ou une solution aqueuse de silicate de métal alcalin contenant éventuellement un électrolyte, éventuellement partiellement neutralisée par un acide, ou une suspension (bouillie) de silice, de pH de l'ordre de 6 à 9,

    en maintenant pendant l'introduction simultanée des réactifs un pH du milieu sensiblement constant de l'ordre de 7 à 9,
    puis en introduisant, éventuellement, un acide jusqu'à obtention d'un pH de l'ordre de 3 à 6.

10. Procédé selon l'une quelconque des revendications 6) à 9), **caractérisé en ce que** l'opération de traitement de la bouillie de silice est réalisée à l'aide d'un composé acide du zinc choisi parmi les sels inorganiques ou organiques solubles du zinc de degré d'oxydation "2".

11. Procédé selon l'une quelconque des revendications 6) à 10), **caractérisé en ce que** l'opération de traitement de la bouillie de silice réalisée à l'aide d'un composé acide du zinc, est suivie ou accompagnée de l'addition d'un agent basique choisi parmi l'ammoniaque, l'hydroxyde de sodium, les silicates alcalins.

**12.** Procédé selon l'une quelconque des revendications 6) à 9), **caractérisé en ce que** l'opération de traitement de la bouillie de silice est réalisée à l'aide d'un composé basique du zinc choisi parmi les zincates contenant les anions $ZnO_2^{2-}$, $HZnO_2^-$, $Zn_2O_4^{4-}$, $ZnO_4^{6-}$.

**13.** Procédé selon l'une quelconque des revendications 6) à 9) ou 12), **caractérisé en ce que** l'opération de traitement de la bouillie de silice réalisée à l'aide d'un composé basique du zinc, est suivie de l'addition d'un agent acide choisi parmi les acides nitrique, sulfurique, chlorhydrique, carbonique.

**14.** Procédé selon l'une quelconque des revendications 6) à 13), **caractérisé en ce que** l'opération de traitement à l'aide d'au moins un composé soluble du zinc et de l'agent basique ou acide de précipitation du dérivé du zinc, est réalisée soit sur la bouillie en cours de précipitation, soit en fin de précipitation et/ou après précipitation, et ce avant l'étape de filtration/lavage.

**15.** Procédé selon l'une quelconque des revendications 6) à 11) ou 14), **caractérisé en ce que** l'opération de traitement par le composé soluble du zinc, est réalisée en ajoutant à une bouillie de silice de pH de l'ordre de 7 à 9, de préférence de l'ordre de 7,5 à 8,5 , en fin et/ou après précipitation à une température de l'ordre de 15 à 95°C, une solution aqueuse de sel acide soluble de zinc de degré d'oxydation "2", puis un agent basique, jusqu'à obtenir un pH de l'ordre de 7,5 à 9,5, de préférence de l'ordre de 7,5 à 9.

**16.** Procédé selon l'une quelconque des revendications 6) à 11) ou 14), **caractérisé en ce que** l'opération de traitement par le composé soluble du zinc, est réalisée en ajoutant à une bouillie de silice en cours de précipitation, de pH de l'ordre de 7 à 9, de préférence de l'ordre de 7,5 à 8,5 , simultanément une solution de silicate et une solution de sel acide de zinc.

**17.** Procédé selon l'une quelconque des revendications 6) à 16), **caractérisé en ce que** l'étape de séparation de la bouillie traitée est réalisé par filtration, et est de préférence couplée avec une opération de lavage.

**18.** Procédé selon la revendication 17), **caractérisé en ce que** les étapes de filtration /lavage de la bouillie traitée sont de préférence réalisées à l'aide d'un filtre presse et d'eau comme agent de lavage, ce afin d'obtenir un gâteau de silice d'extrait sec désiré, d'au moins 15% en poids, de préférence d'au moins 16% en poids.

**19.** Procédé selon l'une quelconque des revendications 6) à 18), **caractérisé en ce que** l'étape de délitage du gâteau est réalisée par simple action d'agitation mécanique à l'aide d'un mobile cisaillant, éventuellement avec ajout d'eau.

**20.** Procédé selon l'une quelconque des revendications 6) à 19), **caractérisé en ce que** l'étape d'atomisation est réalisée à l'aide d'un atomiseur à buse.

**21.** Procédé selon l'une quelconque des revendications 6), 7), 9 à 11), 15), 17 à 20), **caractérisé en ce qu'**on réalise

* une opération de formation de bouillie de silice par réaction d'un silicate de métal alcalin et d'un agent acidifiant, selon les étapes suivantes :

  - une première étape consistant à mettre en oeuvre un pied de cuve initial constitué d'eau, de silicate de métal alcalin et d'un sel électrolyte, la concentration en silicate de métal alcalin (exprimé en $SiO_2$) dans ledit pied de cuve pouvant aller jusqu'à 100 g/l.
  - une deuxième étape consistant à neutraliser ledit pied de cuve par l'agent acidifiant jusqu'à obtention d'un pH du milieu réactionnel supérieur ou égal à environ 7, de préférence de l'ordre de 7 à 9,5, tout particulièrement de l'ordre de 7 à 8,5 ;
  - une troisième étape consistant à introduire dans ledit pied de cuve neutralisé le silicate de métal alcalin en solution aqueuse et l'agent acidifiant, dans des conditions telles que le pH du milieu réactionnel reste sensiblement constant et supérieur ou égal à environ 7, de préférence de l'ordre de 7 à 9,5, tout particulièrement de l'ordre de 7 à 8,5 ;

* une opération de traitement de la bouillie obtenue, après mûrissement éventuel, par addition de 0,5 à 2 parties, de préférence de 0,5 à moins de 2 parties en poids, tout particulièrement de 0,6 à 1,5 partie (exprimée en zinc métal) d'au moins un composé soluble acide du zinc de degré d'oxydation "2" pour 100 parties de silice, puis, après mûrissement éventuel, addition d'un agent basique, ce jusqu'à obtenir un pH du milieu réactionnel de l'ordre de 7,5 à 9,5 , de préférence de l'ordre de 7,5 à 9 et mûrissement éventuel ;

* éventuellement une opération d'ajustement de pH à une valeur de l'ordre de 7 à 8,5 par addition d'un agent acide et mûrissement éventuel ;
* une opération de séparation et de lavage de manière à obtenir un gâteau de silice présentant un extrait sec d'au moins 15% en poids, de préférence d'au moins 16% en poids, notamment à l'aide d'un filtre presse
* une opération de délitage par des moyens d'agitation mécaniques
* et une opération de séchage par atomisation.

22. Procédé de préparation de la silice sous forme de microperles faisant l'objet de la revendication 5), selon l'une quelconque des revendications 6) à 21), **caractérisé en ce qu'**on introduit à un moment quelconque de la synthèse de la bouillie de silice ou de préférence lors de l'étape de délitage du gâteau de silice contenant du zinc, de l'ordre de 0,2 à 5 parties en poids, de préférence de l'ordre de 0,5 à 4 parties en poids pour 100 parties en poids de silice exprimées en $SiO_2$, d'au moins un pigment minéral, blanc notamment.

23. Utilisation de la silice sous forme des microperles faisant l'objet de l'une quelconque des revendications 1) à 5) ou susceptibles d'être obtenues selon le procédé faisant l'objet de l'une quelconque des revendications 6) à 22), comme agent sensoriel en bouche, dans les compositions dentifrices, par désintégration au brossage.

24. Compositions dentifrices comprenant de la silice sous forma de microperles faisant l'objet de l'une quelconque des revendications 1) à 5) ou susceptible d'être obtenue selon le procédé faisant l'objet de l'une quelconque des revendications 6) à 22).

25. Utilisation selon la revendication 23) ou compositions dentifrices selon la revendication 24), caractérisée(s) en ce que la quantité de silice sous forme de microperles représente de l'ordre de 0,5 à 20%, de préférence de l'ordre de 1 à 15% desdites compositions.

**Claims**

1. Fumed silica having:

* a CTAB specific surface of at least 100 m$^2$/g, preferably from about 120 m$^2$/g to 250 m$^2$/g and most particularly from about 140 m$^2$/g to 200 m$^2$/g;
* a DOP oil uptake of at least 200 ml/g, preferably from about 200 ml/g to 350 ml/g;
* at the surface a zinc derivative of oxidation state "2"
* a pH from about 7 to 9, preferably from about 7.5 to 8.7;

the said silica being **characterized**

* **in that** it is in the form of microbeads having a median particle diameter from 50 μm to 600 μm, generally from about 100 μm to 400 μm,
* **in that** the amount of zinc derivative of oxidation state "2" present at the surface ranges from 0.5 to 2 parts by weight, preferably from 0.5 to less than 2 parts by weight, most particularly from 0.6 to 1.5 parts by weight (expressed as zinc metal) per 100 parts by weight of silica ($SiO_2$),
* and **in that** the said microbeads can disintegrate in a toothpaste composition containing them, during a brushing operation, providing a sensory effect in the mouth.

2. Silica according to Claim 1, **characterized in that** it has a BET specific surface of at least 100 m$^2$/g, preferably from about 120 m$^2$/g to 300 m$^2$/g, most particularly from about 140 m$^2$/g to 250 m$^2$/g.

3. Silica according to Claim 1 or 2, **characterized in that** the said microbeads have, for a diameter of 180 μm ± 10μm, a cohesion factor from 50% to less than 90%, preferably from 55% to 80%.

4. Silica according to any one of Claims 1 to 3, **characterized in that** the said microbeads are compatible with fluorinated compounds, their compatibility being greater than 75%, preferably greater than 85%, with NaF.

5. Silica according to any one of Claims 1 to 4, **characterized in that** the said microbeads comprise at least one mineral pigment, in particular a white mineral pigment, in an amount of from about 0.2 to 5 parts by weight, preferably from 0.5 to 4 parts by weight (expressed as weight of pigment), per 100 parts by weight of silica ($SiO_2$).

**6.** Process for preparing fumed silica in the form of microbeads with sensory properties in the mouth forming the subject of any one of Claims 1 to 4, comprising the steps for formation of an aqueous silica slurry by reacting a silicate of an alkali metal M, with an $SiO_2/M_2O$ ratio from about 2 to 4, with an acidifying agent, separation of the silica slurry formed, washing, fluidization (crumbling) of the silica cake recovered and drying, the said process being **characterized in that**:

- the crumbling operation is carried out on a silica cake with a solids content of at least about 15% by weight, the said cake resulting from the separation of a silica slurry treated with from 0.5 to 2 parts by weight, preferably from 0.5 to less than 2 parts by weight, most particularly from 0.6 to 1 part by weight (expressed as zinc metal) of at least one acidic or basic soluble zinc compound with an oxidation state of 2 per 100 parts by weight of silica ($SiO_2$), and performed using a basic agent (in the case of a treatment using at least acidic zinc compound) or acidic agent (in the case of a treatment using at least one basic zinc compound), respectively, at a pH value from about 7.5 to 9.5, preferably from about 7.5 to 9
- and the drying operation is carried out by atomization.

**7.** Process according to Claim 6, **characterized in that** the step for formation of the silica slurry is carried out at a temperature of at least 60°C, preferably from about 70°C to 98°C, most particularly from 80°C to 98°C.

**8.** Process according to Claim 6 or 7, **characterized in that** the step for formation of the slurry is carried out by gradual neutralization of a tail stock consisting of an aqueous alkali metal silicate solution optionally containing an electrolyte, by continuous or batchwise addition of an acid.

**9.** Process according to Claim 6 or 7, **characterized in that** the step for formation of the slurry is carried out by simultaneously introducing an alkali metal silicate solution and an acid onto a tail stock consisting of:

- water optionally with an acid or base added, with a pH from about 4 to 11
- or an aqueous alkali metal silicate solution optionally containing an electrolyte, optionally partially neutralized with an acid, or a silica suspension (slurry), with a pH from about 6 to 9 while maintaining a substantially constant pH from about 7 to 9 in the medium during the simultaneous introduction of the reagents, and then by introducing, optionally, an acid until a pH from about 3 to 6 is obtained.

**10.** Process according to any one of Claims 6 to 9, **characterized in that** the operation for treatment of the silica slurry is carried out using an acidic zinc compound chosen from soluble inorganic or organic zinc salts of oxidation state "2".

**11.** Process according to any one of Claims 6 to 10, **characterized in that** the operation for treatment of the silica slurry carried out using an acidic zinc compound is followed or accompanied by the addition of a basic agent chosen from aqueous ammonia, sodium hydroxide and alkaline silicates.

**12.** Process according to any one of Claims 6 to 9, **characterized in that** the operation for treatment of the silica slurry is carried out using a basic zinc compound chosen from zincates containing $ZnO_2^{2-}$, $HZnO_2^-$, $Zn_2O_4^{4-}$ and $ZnO_4^{6-}$ anions.

**13.** Process according to any one of Claims 6 to 9 or 12, **characterized in that** the operation for treatment of the silica slurry carried out using a basic zinc compound is followed by addition of an acidic agent chosen from nitric acid, sulphuric acid, hydrochloric acid and carbonic acid.

**14.** Process according to any one of Claims 6 to 13, **characterized in that** the treatment operation using at least one soluble zinc compound and the basic or acidic agent for precipitating the zinc derivative is carried out either on the slurry during precipitation or at the end of precipitation and/or after precipitation, before the filtration/washing step.

**15.** Process according to any one of Claims 6 to 11 or 14, **characterized in that** the treatment operation with the soluble zinc compound is carried out by adding to a silica slurry with a pH from about 7 to 9, preferably from about 7.5 to 8.5, at the end and/or after precipitation at a temperature from about 15°C to 95°C, an aqueous solution of a soluble acidic zinc salt of oxidation state "2", followed by a basic agent, until a pH from about 7.5 to 9.5, preferably from about 7.5 to 9, is obtained.

**16.** Process according to any one of Claims 6 to 11 or 14, **characterized in that** the treatment operation with the soluble zinc compound is carried out by simultaneously adding to a silica slurry during precipitation, with a pH from about 7 to 9, preferably from about 7.5 to 8.5, a silicate solution and an acidic zinc salt solution.

**17.** Process according to any one of Claims 6 to 16, **characterized in that** the step for separation of the treated slurry is carried out by filtration and is preferably coupled with a washing operation.

**18.** Process according to Claim 17, **characterized in that** the steps of filtration/washing of the treated slurry are preferably carried out using a filter press and water as washing agent, in order to obtain a silica cake of the desired solids content, of at least 15% by weight, preferably of at least 16% by weight.

**19.** Process according to any one of Claims 6 to 18, **characterized in that** the step of crumbling of the cake is carried out by the simple action of mechanical stirring using a shearing rotor, optionally with addition of water.

**20.** Process according to any one of Claims 6 to 19, **characterized in that** the atomization step is carried out using a nozzle sprayer.

**21.** Process according to any one of Claims 6, 7, 9 to 11, 15 and 17 to 20, **characterized in that** the following are carried out

* an operation for formation of silica slurry by reaction of an alkali metal silicate and an acidifying agent, according to the following steps:

  - a first step consisting in using an initial tail stock consisting of water, alkali metal silicate and an electrolytic salt, the concentration of alkali metal silicate (expressed as $SiO_2$) in the said tail stock possibly being up to 100 g/l
  - a second step consisting in neutralizing the said tail stock with the acidifying agent until a pH of greater than or equal to about 7, preferably from about 7 to 9.5 and most particularly from about 7 to 8.5, is obtained in the reaction medium;
  - a third step consisting in introducing into the said neutralized tail stock the alkali metal silicate in aqueous solution and the acidifying agent, under conditions such that the pH of the reaction medium remains substantially constant and above or equal to about 7, preferably from about 7 to 9.5 and most particularly from about 7 to 8.5;

* an operation for treatment of the slurry obtained, after optional maturation, by addition of 0.5 to 2 parts, preferably from 0.5 to less than 2 parts by weight and most particularly from 0.6 to 1.5 parts (expressed as zinc metal), of at least one soluble acidic zinc compound of oxidation state "2" per 100 parts of silica, and then, after optional maturation, addition of a basic agent, until a pH from about 7.5 to 9.5, preferably from about 7.5 to 9, is obtained in the reaction medium, and optional maturation;
* optionally, an operation to adjust the pH to a value from about 7 to 8.5 by addition of an acidic agent, and optional maturation;
* an operation of separation and washing so as to obtain a silica cake with a solids content of at least 15% by weight, preferably of at least 16% by weight, in particular using a filter press;
* an operation for crumbling by means of mechanical stirrers;
* and a spray-drying operation.

**22.** Process for preparing the silica in the form of microbeads forming the subject of Claim 5, according to any one of Claims 6 to 21, **characterized in that** from about 0.2 to 5 parts by weight, preferably from about 0.5 to 4 parts by weight, per 100 parts by weight of silica, expressed as $SiO_2$, of at least one mineral pigment, in particular a white mineral pigment, are introduced at any point in the synthesis of the silica slurry or, preferably, during the step of crumbling of the silica cake containing zinc.

**23.** Use of the silica in the form of microbeads forming the subject of any one of Claims 1 to 5 or which can be obtained according to the process forming the subject of any one of Claims 6 to 22, as sensory agent in the mouth, in toothpaste compositions by disintegration on brushing.

**24.** Toothpaste compositions comprising silica in the form of microbeads forming the subject of any one of Claims 1 to 5 or which can be obtained according to the process forming the subject of any one of Claims 6 to 22.

**25.** Use according to Claim 23 or toothpaste compositions according to Claim 24, **characterized in that** the amount of silica in the form of microbeads represents from about 0.5% to 20%, preferably from about 1% to 15%, of the said compositions.

**Patentansprüche**

**1.** Fällungskieselsäure, aufweisend:

- eine spezifische CTAB-Oberfläche von mindestens 100 m$^2$/g, vorzugsweise in der Größenordnung von 120 bis 250 m$^2$/g, insbesondere in der Größenordnung von 140 bis 200 m$^2$/g;

- eine Ölzahl DOP von mindestens 200 ml/g, vorzugsweise in der Grössenordnung von 200 bis 350 ml/g;

- an der Oberfläche eine Zinkderivat mit einem Oxidationsgrad von "2";

- einen pH-Wert in der Größenordnung von 7 bis 9, vorzugsweise in der Größenordnung von 7,5 bis 8,7;

**dadurch gekennzeichnet,**

- **daß** sie in Form von Mikrokugeln mit einem mittleren Teilchendurchmesser von 50 bis 600 µm, im allgemeinen in der Größenordnung von 100 bis 400 µm, vorliegt,

- **daß** die Menge des an der Oberfläche vorhandenen Zinkderivats mit einem Oxidationsgrad von "2" 0,5 bis 2 Gewichtsteile, vorzugsweise 0,5 bis weniger als 2 Gewichtsteile, insbesondere 0,6 bis 1,5 Gewichtsteile, berechnet als Zinkmetall, auf 100 Gewichtsteile Siliciumdioxid (SiO$_2$) beträgt und

- **daß** die Mikrokugeln sich in einer sie enthaltenden Zahnpflegemittelzusammensetzung bei einem Putzvorgang zersetzen können, was einen sensorischen Effekt im Mund bewirkt.

**2.** Kieselsäure nach Anspruch 1, **dadurch gekennzeichnet, daß** sie eine spezifische BET-Oberfläche von mindestens 100 m$^2$/g, vorzugsweise in der Größenordnung von 120 bis 300 m$^2$/g, insbesondere in der Größenordnung von 140 bis 250 m$^2$/g, aufweist.

**3.** Kieselsäure nach Anspruch 1 oder 2, **dadurch gekennzeichnet, daß** die Mikrokugeln bei einem Durchmesser von 180 µm ± 10 µm einen Kohäsionsfaktor im Bereich von 50 bis weniger als 90 %, vorzugsweise von 55 bis 80 %, aufweisen.

**4.** Kieselsäure nach einem der Ansprüche 1 bis 3, **dadurch gekennzeichnet, daß** die Mikrokugeln mit fluorierten Verbindungen kompatibel sind, wobei ihre Kompatibilität mit NaF größer als 75 %, vorzugsweise größer als 85 %, ist.

**5.** Kieselsäure nach einem der Ansprüche 1 bis 4, **dadurch gekennzeichnet, daß** die Mikrokugeln mindestens ein anorganisches, vorzugsweise weißes Pigment in einer Menge in der Größenordnung von 0,2 bis 5 Gewichtsteilen, vorzugsweise 0,5 bis 4 Gewichtsteilen, berechnet als Pigmentgewicht, auf 100 Gewichtsteile Siliciumdioxid (SiO$_2$) umfassen.

**6.** Verfahren zur Herstellung einer Fällungskieselsäure in Form von Mikrokugeln mit sensorischen Eigenschaften im Mund nach einem der Ansprüche 1 bis 4, umfassend die Verfahrensschritte der Bildung eines wäßrigen Kieselsäureschlickers durch Reaktion eines Silikats eines Alkalimetalls M mit einem SiO$_2$/M$_2$O-Verhältnis in der Größenordnung von 2 bis 4 mit einem sauermachenden Mittel, der Abtrennung des gebildeten Kieselsäureschlikkers, des Waschens, der Verflüssigung (Zerkleinerung) des erhaltenen Kieselsäurekuchens und der Trocknung, **dadurch gekennzeichnet, daß**

- der Zerkleinerungsvorgang an einem Kieselsäurekuchen mit einem Trockenextrakt von mindestens etwa 15 Gew.-% durchgeführt wird, wobei der Kuchen aus der Abtrennung eines Kieselsäureschlickers resultiert, der mittels 0,5 bis 2 Gewichtsteilen, vorzugsweise 0,5 bis weniger als 2 Gewichtsteilen, insbesondere 0,6 bis 1 Gewichtsteil, berechnet als Zinkmetall, mindestens einer sauren oder basischen löslichen Zinkverbindung mit einem Oxidationsgrad 2 auf 100 Gewichtsteile Siliciumdioxid ($SiO_2$) behandelt und mittels eines basischen Reagenzes (im Fall einer Behandlung mittels mindestens einer sauren Zinkverbindung) bzw. eines sauren Reagenzes (im Fall einer Behandlung mittels einer basischen Zinkverbindung) auf einen pH-Wert in der Größenordnung von 7,5 bis 9,5, vorzugsweise in der Größenordnung von 7,5 bis 9, gebracht worden ist und

-   der Vorgang der Trocknung durch Zerstäubung durchgeführt wird.

7.  Verfahren nach Anspruch 6, **dadurch gekennzeichnet, daß** der Verfahrensschritt der Bildung des Kieselsäureschlickers bei einer Temperatur von mindestens 60 °C, vorzugsweise in der Größenordnung von 70 bis 98 °C, insbesondere 80 bis 98 °C, durchgeführt wird.

8.  Verfahren nach Anspruch 6 oder 7, **dadurch gekennzeichnet, daß** der Verfahrensschritt der Bildung des Schlickers durch allmähliche Neutralisation einer Vorlage, die aus einer wäßrigen Alkalimetallsilikatlösung besteht, die gegebenenfalls einen Elektrolyten enthält, durch kontinuierliche oder diskontinuierliche Zugabe einer Säure durchgeführt wird.

9.  Verfahren nach Anspruch 6 oder 7, **dadurch gekennzeichnet, daß** der Verfahrensschritt der Bildung des Schlickers durchgeführt wird, indem man gleichzeitig eine Alkalimetallsilikatlösung und eine Säure einbringt in die Vorlage, bestehend aus

-   Wasser, dem gegebenenfalls eine Säure oder Base zugesetzt ist, mit einem pH-Wert in der Größenordnung von 4 bis 11 oder

-   einer wäßrigen Alkalimetallsilikatlösung, enthaltend gegebenenfalls einen Elektrolyten, der gegebenenfalls teilweise mit einer Säure neutralisiert ist, oder eine Kieselsäuresuspension (Schlicker) mit einem pH-Wert in der Größenordnung von 6 bis 9,

wobei man während der gleichzeitigen Zugabe der Reagenzien einen pH-Wert des Milieus in etwa konstanthält in der Größenordnung von 7 bis 9,
man dann gegebenenfalls eine Säure hinzugibt, bis man einen pH-Wert in der Größenordnung von 3 bis 6 erhält.

10. Verfahren nach einem der Ansprüche 6 bis 9, **dadurch gekennzeichnet, daß** der Vorgang der Behandlung des Kieselsäureschlickers mittels einer sauren Zinkverbindung durchgeführt wird, die ausgewählt ist aus löslichen, organischen oder anorganischen Zinksalzen mit einem Oxidationsgrad von "2".

11. Verfahren nach einem der Ansprüche 6 bis 10, **dadurch gekennzeichnet, daß** der Vorgang der Behandlung des Kieselsäureschlickers, der mittels einer sauren Zinkverbindung durchgeführt wird, gefolgt oder begleitet wird von der Zugabe eines basischen Reagenzes, ausgewählt aus Ammoniak, Natriumhydroxid und Alkalisilikaten.

12. Verfahren nach einem der Ansprüche 6 bis 9, **dadurch gekennzeichnet, daß** der Vorgang der Behandlung des Kieselsäureschlickers mittels einer basischen Zinkverbindung durchgeführt wird, ausgewählt aus Zinkaten, welche die Anionen $ZnO_2^{2-}$, $HZnO_2^-$, $Zn_2O_4^{4-}$ oder $ZnO_4^{6-}$ enthalten.

13. Verfahren nach einem der Ansprüche 6 bis 9 oder 12, **dadurch gekennzeichnet, daß** der Vorgang der Behandlung des Kieselsäureschlickers, der mittels einer basischen Zinkverbindung durchgeführt wird, von einer Zugabe eines sauren Reagenzes gefolgt wird, das ausgewählt ist aus Salpetersäure, Schwefelsäure, Salzsäure oder Kohlensäure.

14. Verfahren nach einem der Ansprüche 6 bis 13, **dadurch gekennzeichnet, daß** der Vorgang der Behandlung mittels mindestens einer löslichen Zinkverbindung und eines basischen oder sauren Fällungsreagenzes des Zinkderivats entweder an dem Schlicker im Verlauf der Fällung oder am Ende der Fällung und/oder nach der Fällung durchgeführt wird, und dies vor dem Verfahrensschritt des Filtrierens/Waschens.

15. Verfahren nach einem der Ansprüche 6 bis 11 oder 14, **dadurch gekennzeichnet, daß** der Vorgang der Behandlung mittels der löslichen Zinkverbindung durchgeführt wird, indem man zu einem Kieselsäureschlicker mit einem pH-Wert in der Größenordnung von 7 bis 9, vorzugsweise in der Größenordnung von 7,5 bis 8,5, am Ende und/oder nach der Fällung bei einer Temperatur in der Größenordnung von 15 bis 95 °C eine wäßrige Lösung eines löslichen, sauren Zinksalzes mit einem Oxidationsgrad von "2", dann ein basisches Reagenz hinzugibt, bis man einen pH-Wert in der Größenordnung von 7,5 bis 9,5, vorzugsweise in der Größenordnung von 7,5 bis 9, erhält.

16. Verfahren nach einem der Ansprüche 6 bis 11 oder 14, **dadurch gekennzeichnet, daß** der Vorgang der Behandlung mittels der löslichen Zinkverbindung durchgeführt wird, indem man zu einem Kieselsäureschlicker mit einem pH-Wert in der Größenordnung von 7 bis 9, vorzugsweise in der Größenordnung von 7,5 bis 8,5, im Verlauf der Fällung gleichzeitig eine Silikatlösung und eine Lösung eines sauren Zinksalzes hinzugibt

17. Verfahren nach einem der Ansprüche 6 bis 16, **dadurch gekennzeichnet, daß** der Verfahrensschritt der Abtrennung des behandelten Schlickers durch Filtrieren durchgeführt wird, vorzugsweise zusammen mit einem Waschvorgang.

18. Verfahren nach Anspruch 17, **dadurch gekennzeichnet, daß** die Verfahrensschritte Filtrieren/Waschen des behandelten Schlickers vorzugsweise mittels einer Filterpresse und Wasser als Waschreagenz durchgeführt wird, um einen Kieselsäurekuchen mit einem gewünschten Trockenextrakt von mindestens 15 Gew.-%, vorzugsweise mindestens 16 Gew.-%, zu erhalten.

19. Verfahren nach einem der Ansprüche 6 bis 18, **dadurch gekennzeichnet, daß** der Verfahrensschritt des Zerkleinerns des Kuchens durch einfache Einwirkung mechanischer Bewegung mittels einer Schervorrichtung, gegebenenfalls unter Zugabe von Wasser, durchgeführt wird.

20. Verfahren nach einem der Ansprüche 6 bis 19, **dadurch gekennzeichnet, daß** der Verfahrensschritt des Zerstäubens mittels eines Zerstäubers mit Düse durchgeführt wird.

21. Verfahren nach einem der Ansprüche 6, 7, 9 bis 11, 15, 17 bis 20, **dadurch gekennzeichnet, daß** man durchführt:

- einen Vorgang der Bildung des Kieselsäureschlickers durch Reaktion eines Alkalimetallsilikats und eines sauermachenden Mittels entsprechend den folgenden Verfahrensschritten:

  - einem ersten Verfahrensschritt, bei dem man eine anfängliche Vorlage einsetzt, die aus Wasser, einem Alkalimetallsilikat und einem Elektrolytsalz besteht, wobei die Konzentration an Alkalimetallsilikat, berechnet als $SiO_2$, in der Vorlage bis zu 100 g/l erreichen kann,

  - einem zweiten Verfahrensschritt, bei dem man die Vorlage mit einem sauermachenden Reagenz neutralisiert, bis man einen pH-Wert des Reaktionsmilieus von mehr als oder gleich etwa 7, vorzugsweise in der Größenordnung von 7 bis 9,5, insbesondere in der Größenordnung von 7 bis 8,5, erhält,

  - einem dritten Verfahrensschritt, bei dem man zu der neutralisierten Vorlage das Alkalimetallsilikat in wäßriger Lösung und das sauermachende Mittel unter solchen Bedingungen hinzugibt, daß der pH-Wert des Reaktionsmilieus in etwa konstantbleibt und größer oder gleich etwa 7, vorzugsweise in der Größenordnung von 7 bis 9,5, insbesondere in der Größenordnung von 7 bis 8,5, ist,

- einen Vorgang der Behandlung des erhaltenen Schlickers, gegebenenfalls nach Reifung, durch Zugabe von 0,5 bis 2 Gewichtsteilen, vorzugsweise 0,5 bis weniger als 2 Gewichtsteilen, insbesondere 0,6 bis 1,5 Gewichtsteilen, berechnet als Zinkmetall, mindestens einer löslichen, sauren Zinkverbindung mit einem Oxidationsgrad von "2" auf 100 Teile Siliziumdioxid, dann, gegebenenfalls nach Reifung, der Zugabe eines basischen Reagenzes, bis man einen pH-Wert des Reaktionsmilieus in der Größenordnung von 7,5 bis 9,5, vorzugsweise in der Größenordnung von 7,5 bis 9, erhält, und gegebenenfalls der Reifung,

- gegebenenfalls einen Vorgang der pH-Wert-Anpassung auf einen Wert in der Größenordnung von 7 bis 8,5 durch Zugabe eines sauren Reagenzes und gegebenenfalls der Reifung,

- einen Vorgang der Abtrennung und des Waschens, so daß man einen Kieselsäurekuchen mit einem Trockenextrakt von mindestens 15 Gew.-%, vorzugsweise mindestens 16 Gew.-%, insbesondere mit Hilfe einer Filterpresse, erhält,

- einen Vorgang des Zerkleinerns mit Hilfe mechanischer Mittel und

- einen Vorgang der Trocknung durch Zerstäuben.

22. Verfahren zur Herstellung der Kieselsäure in Form von Mikrokugeln, die Gegenstand des Anspruchs 5 ist, nach einem der Ansprüche 6 bis 21, **dadurch gekennzeichnet, daß** man zu einem beliebigen Zeitpunkt der Synthese des Kieselsäureschlickers, vorzugsweise während des Verfahrensschritts des Zerkleinerns des zinkhaltigen Kieselsäurekuchens, etwa 0,2 bis 5 Gewichtsteile, vorzugsweise etwa 0,5 bis 4 Gewichtsteile, auf 100 Gewichtsteile der Kieselsäure, berechnet als $SiO_2$, mindestens eines anorganischen, insbesondere weißen Pigments hinzugibt.

23. Verwendung der Kieselsäure in Form von Mikrokugeln nach einem der Ansprüche 1 bis 5 oder erhältlich nach dem Verfahren nach einem der Ansprüche 6 bis 22 als im Mund sensorisch wirkendes Mittel in Zahnpflegemittelzusammensetzungen durch Zersetzung beim Putzen.

24. Zahnpflegemittelzusammensetzungen, enthaltend die Kieselsäure in Form von Mikrokugeln nach einem der Ansprüche 1 bis 5 oder erhältlich durch das Verfahren nach einem der Ansprüche 6 bis 22.

25. Verwendung nach Anspruch 23 oder Zahnpflegemittelzusammensetzungen nach Anspruch 24, **dadurch gekennzeichnet, daß** die Menge an Kieselsäure in Form von Mikrokugeln etwa 0,5 bis 20 %, vorzugsweise etwa 1 bis 15 %, dieser Zusammensetzungen darstellt.